(19) Europäisches Patentamt
European Patent Office
Office européen des brevets

(11) Publication number: **0 070 638**
**B1**

# (12) EUROPEAN PATENT SPECIFICATION

(45) Date of publication of patent specification: **20.03.85**

(51) Int. Cl.$^4$: **C 07 F 1/00**, C 07 F 1/08, C 07 C 7/152

(21) Application number: **82303471.5**

(22) Date of filing: **01.07.82**

## (54) Copper or silver complexes with fluorinated diketonates and unsaturated hydrocarbons.

(30) Priority: **13.07.81 US 282652**
**13.07.81 US 282653**

(43) Date of publication of application:
**26.01.83 Bulletin 83/04**

(45) Publication of the grant of the patent:
**20.03.85 Bulletin 85/12**

(84) Designated Contracting States:
**DE FR GB**

(56) References cited:
**US-A-3 755 487**

**INORGANIC SYNTHESIS, vol. 16, no. 33, 1976, pages 117-19; W. PARTENHEIMER et al.: "Olefin (beta-diketonato)silver(I) compounds"**

**JOURNAL OF THE CHEMICAL SOCIETY, DALTON, 1972, pages 2208-13; R.C.E. BELFORD et al.: "Reactions of 1,4-diazabicyclo (2,2,2)octane with Bis-(1,1,1,5,5,5-hexafluoropentan e-2,4-dionato)copper (II) and the crystal structure of the 1:1 complex"**

(73) Proprietor: **Exxon Research and Engineering Company**
**P.O.Box 390 180 Park Avenue**
**Florham Park New Jersey 07932 (US)**

(72) Inventor: **Doyle, Gerald**
**Box 20 RD 1**
**Whitehouse Station New Jersey (US)**

(74) Representative: **Field, Roger Norton et al**
**ESSO Engineering (Europe) Ltd. Patents & Licences Apex Tower High Street**
**New Malden Surrey KT3 4DJ (GB)**

(56) References cited:
**Inorganic Chemistry, Vol. 11, page 2840-2841, 1972**

**EP 0 070 638 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to the formation of new copper or silver complexes containing a fluorinated diketonate and unsaturated hydrocarbons as ligands. More particularly, copper or silver in the +1 formal oxidation state form complexes with fluorinated beta-diketonates and unsaturated ligands containing at least one non-aromatic unsaturation, and the reaction to form the complexes may be used to remove unsaturated hydrocarbons from feedstreams.

It is known that certain silver (I) and copper (I) salts form complexes with olefins and acetylenes. For example, cuprous chloride is known to form complexes with both ethylene and acetylene. U.S. Patent No. 3,401,112 teaches a method of separating a mixture of hydrocarbons having differing degrees of unsaturation using a copper (I) salt of the formula CuXA where XA is an anion, X is oxygen or fluorine and A is the remainder of the anion. Examples of fluorinated anions include fluoro substituted carboxylates, fluorosulphonate, perfluoroborate, hexafluorophosphate and hexafluoroantimonate. CuXA forms a cuprous complex with said unsaturated hydrocarbon. Similarly, U.S. Patent No. 3,517,079 describes a process for separating vinyl aromatic hydrocarbons from alkyl aromatic hydrocarbons using a cuprous fluoroborate or cuprous fluorophosphate salt wherein a complex is formed. U.S. Patent Nos. 3,754,047 and 3,755,487 disclose a process for separating complexible ligands such as olefins, acetylenes, aromatics and CO from a feedstream using cuprous salts such as $CuAlCl_4$, $CuBF_4$, $CuOOCCF_3$, $CuPF_6$ and the like. U.S. Patent No. 3,700,416 describes a process for the extraction of metal values from aqueous acidic solutions using fluorinated β-diketonates of the formula

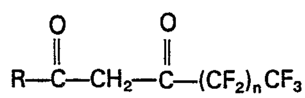

and an organic phase. Examples 2 and 10 show the recovery of copper in the cupric state from a pH 2 acid solution in the presence of an organic phase. U.S. Patent No. 4,279,874 describes the selective removal of CO from gas streams using an absorbent solution containing the reaction product of a Cu(I) compound and a halogenated acetylacetonate of the formula

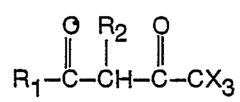 .

The gas streams from which CO is removed may contain alkenes and alkynes in addition to other gases such as $N_2$, $O_2$ and the like. The Cu(I) containing reaction products react with CO to yield a CO-containing complex. *J. C. S. Dalton 2208 (1972)* teaches the reaction of $(hfac)_2Cu(H_2O)$ with $N(CH_2CH_2)_3N$ to form the complex $(hfac)_2Cu$ $(N(CH_2CH_2)_3N)$. The copper is in the 2+ state. *J. C. S. Dalton 984 (1980)* discloses Cu(II) complexes of the type Cu(β-diketonate)′(β-diketonate)″(L) where the β-diketonates may be fluorinated and L is o-phenanthroline, 2,2′-bipyridine or N,N,N′,N′-tetramethylethane-1,2-diamine.

Also Inorganic Chemistry, Volume 11 page 2840-1 (1972) discloses trifluoro- and hexafluoroacetylacetone silver (I) complexes with certain cyclic olefin ligands as prepared by an aqueous process. By contrast in the processes of our invention the presence of an inert organic solvent is necessary to avoid metal disproportionation problems.

It has been discovered that copper (I) and silver (I) can form a new class of complexes with fluorinated acetylacetonate anions and unsaturated hydrocarbons as ligands. The complexes of the invention have the formula

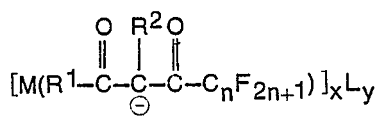

where M is Cu(I) or Ag(I); $R^1$ is $C_1$—$C_6$ fluoroalkyl, $C_1$—$C_8$ alkyl, $C_4$—$C_6$ heterocycle containing O, S or N or $C_6$—$C_{10}$ aryl; $R^2$ is H or $C_1$—$C_6$alkyl with the proviso that $R^1$ and $R^2$ together with the carbons to which they are attached may be joined together to form a $C_6$ ring or a 1,7,7-trimethyl-2-oxobicyclo[2,2,1]hept-3-yl radical; L is an unsaturated hydrocarbon containing at least one ethylenic, acetylenic or isonitrilic unsaturation capable of forming a Cu-L bond, x and y are 1 or 2; and n is an integer from 1 to 8 provided that when M is Ag, if L is an olefin, said olefin must be a linear olefin.

The present invention is also concerned with a process wherein unsaturated hydrocarbons containing at least one ethylenic, acetylenic or isonitrilic unsaturation can be removed from feedstreams by a process which comprises contacting the feedstream with $Cu_2O$ or $Ag_2O$ and a fluorinated acetylacetonate of the formula

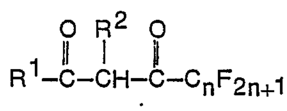

where $R^1$ is $C_1$—$C_6$ fluoroalkyl, $C_1$—$C_8$ alkyl, $C_4$—$C_6$ heterocycle containing O, S or N or $C_6$—$C_{10}$ aryl, $R^2$ is H or $C_1$—$C_6$ alkyl with the proviso that $R^1$ and $R^2$ together with the carbons to which they are attached may be joined together to form a $C_6$ ring or a 1,7,7-trimethyl 1-2 oxobicyclo[2,2,1]hept-3-yl radical and n is an integer from 1 to 8, in an inert organic solvent.

In another embodiment, unsaturated hydrocarbons containing at least one ethylenic, acetylenic or isonitrilic unsaturation can be removed from feedstreams by a process which comprises contacting the feedstream with Cu metal and Cu(II) fluorinated acetylacetonate of the formula

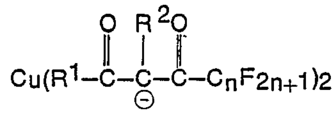

wherein $R^1$ is $C_1$—$C_6$ fluoroalkyl, $C_1$—$C_8$ alkyl, $C_4$—$C_6$ heterocycle containing O, S or N or $C_6$—$C_{10}$ aryl, $R^2$ is H or $C_1$—$C_6$ alkyl with the proviso that $R^1$ and $R^2$ together with the carbons to which they are attached may be joined together to form a $C_6$ ring or a 1,7,7-trimethyl 1-2-oxobicyclo[2,2,1]hept-3-yl radical, and n is an integer from 1 to 8, in an inert organic solvent.

Alternatively, the above-defined unsaturated hydrocarbons can be removed from feedstreams by contacting the feedstreams with a Ag(I) fluorinated acetylacetonate of the formula

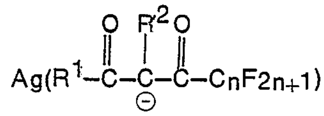

where $R^1$, $R^2$ and n are defined as above, in an inert organic solvent.

Complexes of the formula

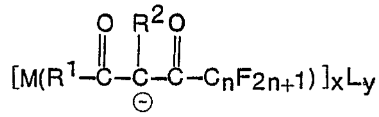

are prepared by reacting metal oxide, fluorinated acetylacetonate and the above-defined unsaturated hydrocarbon in the presence of an inert organic solvent, and this reaction forms the basis for removal of unsaturated hydrocarbons from gas streams. The reaction is illustrated as follows:

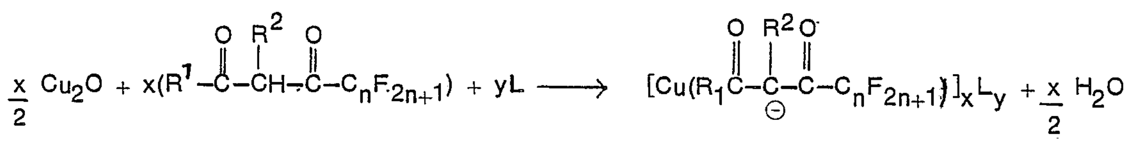

Silver (I) complexes are similarly prepared. Reactants are preferably combined in approximately stoichiometric amounts. The amounts, however, are not critical and variations therefrom are possible. Preferred solvents are ethers, ketones, esters, alcohols, saturated aliphatic hydrocarbons and aromatic hydrocarbons.

When 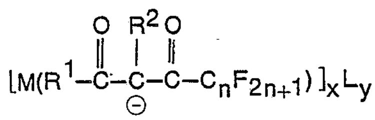

is heated in solution, the following equilibria for the respective metals M are established:

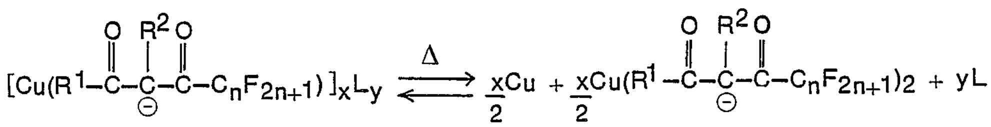

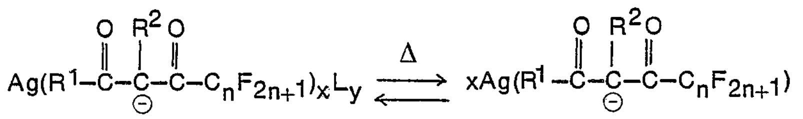

Based on these equilibria, it should also be possible to remove unsaturated hydrocarbons from feedstreams by employing Cu metal plus a Cu(II) fluorinate acetylacetonate or a Ag(I) fluorinated acetylacetonate, and this can be verified experimentally. These equilibrium reactions may also be used to prepare the Cu(I) and Ag(I) complexes of the present invention.

Preferred fluorinated acetylacetonate anion ligands have the formula

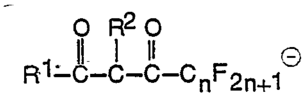

where $R^1$ is $C_1$—$C_3$ fluoroalkyl, especially $CF_3$, $C_1$—$C_6$ alkyl, $C_6$—$C_{10}$ aryl or $C_4$—$C_5$ heterocycle containing O, S or N, $R^2$ is H with the proviso that $R^1$ and $R^2$ may join together to form a $C_6$ ring and n is 1 to 4, especially 1. Examples of preferred embodiments of fluorinated acetylacetonates from which fluorinated acetylacetonate anions are derived include

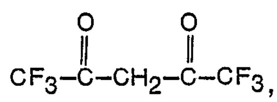

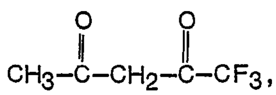 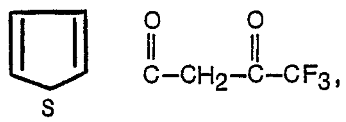 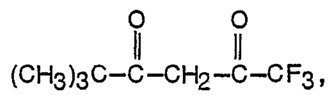

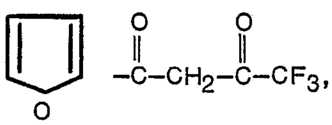 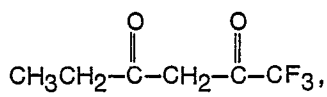 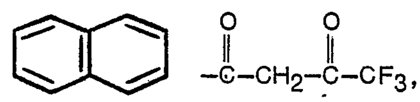

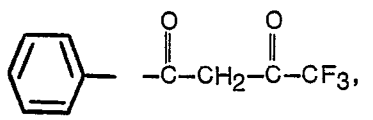 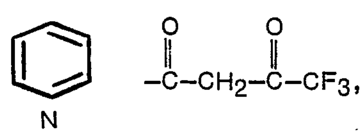

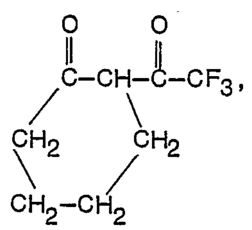

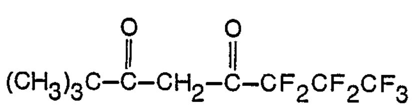 and 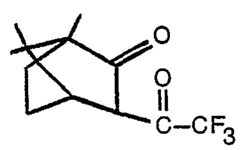 and 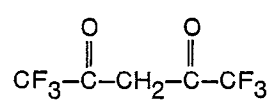

is especially preferred.

Preferred unsaturated hydrocarbons are (a) alkenes of the formula

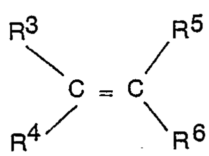

where each $R^3$—$R^6$ is independently H; $C_1$—$C_{30}$, more preferably $C_1$—$C_{15}$ and especially $C_1$—$C_8$ aliphatic with the proviso that any combination of $R^3$, $R^4$, $R^5$ and $R^6$ may be joined together to form at least one $C_4$—$C_{14}$, more preferably $C_5$—$C_{12}$, most preferably $C_6$—$C_8$ cycloaliphatic ring: —C≡N; $C_6$—$C_{10}$ aryl; $C_7$—$C_{14}$ araliphatic;

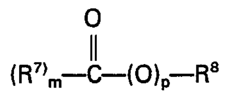

where m and p are 0 or 1, $R^7$ is $C_1$—$C_{20}$, preferably $C_1$—$C_{10}$ aliphatic, and $R^8$ is H, $C_1$—$C_{10}$ aliphatic or $C_6$—$C_{10}$ aryl with the proviso that adjacent

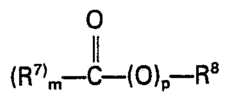

may be joined together to form a $C_4$—$C_{16}$ anhydride; (b) alkynes of the formula $R^9$—C≡C—$R^{10}$ where $R^9$ and $R^{10}$ are independently H; $C_1$—$C_{30}$, more preferably $C_1$—$C_{15}$ and especially $C_1$—$C_8$ aliphatic; $C_6$—$C_{10}$ aryl or $C_7$—$C_{14}$ araliphatic; or (c) isonitriles of the formula $R^{11}$—N≡C where $R^{11}$ is $C_1$—$C_{20}$ aliphatic; $C_3$—$C_{10}$ cycloaliphatic; $C_7$—$C_{20}$ araliphatic or $C_6$—$C_{10}$ aryl. The unsaturated hydrocarbons may be substituted with unreactive substituents such as halogen, cyano, alkoxy, nitro and the like.

Examples of suitable unsaturated ligands include: ethylene, acetylene, 1-octene, isobutylene, 1,5-cyclooctadiene, stilbene, diphenylacetylene, styrene, cyclooctene, 1,5,9-cyclododecatriene, 1,3-hexadiene, isopropylacetylene, 1-decene, 1,5-bicyclophetadiene, 1-octadecene, cyclopentene, octalin, methylene cyclohexane, diphenyl fulvene, 1-octadecyne, benzyl cinnamate, benzal acetophenone, acrolein, acrylonitrile, maleic anhydride, oleic acid, linolenic acid, acrylic acid, methyl methacrylate and diethyl maleate. Suitable isonitriles are, e.g., methyl isocyanide, butyl isocyanide, cyclohexyl isocyanide, phenylethyl isocyanide and phenyl isocyanide.

Examples of copper (I) and silver (I) complexes formed by unsaturated hydrocarbon removal from gas streams are as follows:

Cu(1,5-cyclooctadiene) 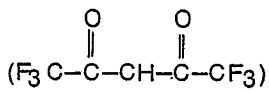

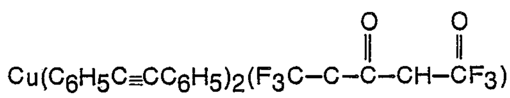

Cu₂(2,5-bicycloheptadiene) 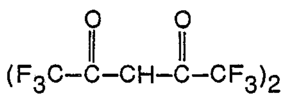

Ag(1-decene) 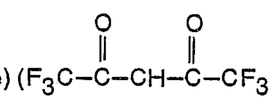

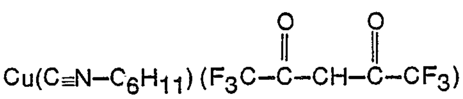

Cu(1,5-cyclooctadiene) 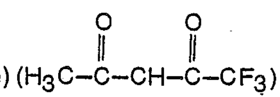

Cu(cyclooctene) 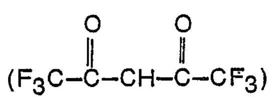

Ag₂(2,5-bicycloheptadiene) 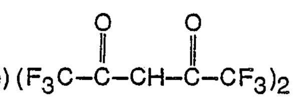

Cu(1,5-cyclooctadiene) 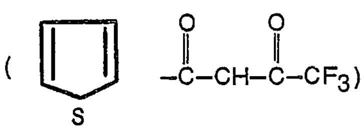

The process for removing the above-defined unsaturated hydrocarbons from feedstreams and for preparing the present Ag(I) or Cu(I) complexes takes place in an inert organic solvent. Preferred solvents are ethers, ketones, esters, alcohols, saturated aliphatic hydrocarbons and aromatic hydrocarbons. It is preferably that the amounts of CO in the reaction mixture should not exceed about 10 vol.%. CO competes with unsaturated hydrocarbon in the formation of cuprous complex and based on thermodynamic considerations, a CO complex forms in preference to the unsaturated hydrocarbon complex as long as competing amounts of CO are present. It is also desirable to carry the reactions in an inert atmosphere, since gases such as oxygen may result in the oxidation of Cu(I) to Cu(II).

Reaction times are not critical. Generally, the reaction mixture is stirred until a clear solution is obtained. A solid product may then be isolated by evaporating solvent. Suitable temperatures are from −100°C to +100°C with room temperature being preferred. If the reaction mixture is heated excessively, it is possible that a dissociative reaction may take place. Thus, copper (I) ethylene complexes are rather unstable due to a high dissociative pressure and heating would not be desirable. On the other hand, higher molecular weight olefins result in stable compounds and the reaction mixture can be heated without harmful results with respect to the removal of unsaturated hydrocarbon.

$Cu_2O$ and/or $Ag_2O$ and fluorinated acetylacetonate are usually present in the reaction mixture in approximately stoichiometric amounts. Wide variations, however, are possible. The concentrations of

metal oxide and fluorinated acetylacetonate may range from 0.001 to 5 M, preferably 0.1 to 3 M. The amount of Cu metal and Cu(II) fluorinated acetylacetonate or Ag(I) fluorinated acetylacetonate may be from 0.001 to 5 M. The feedstream containing the above-defined unsaturated hydrocarbons is contacted with the reaction mixture in either a batchwise or continuous mode. In the case of a gaseous feedstream, the gas may be introduced into the reaction mixture through a gas dispersion device. Liquid feedstreams may be conducted to a stirred reaction vessel or the separation process may be carried out in a countercurrent extractor.

A continuous mode of operation is preferred. Using any of the separation reactions described hereinbefore, a solution is formed containing the complex

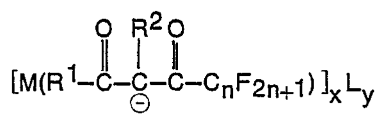

where M, $R^1$, $R^2$, L, x, y and n are as defined above in the separation reactions. This solution is subjected to heating at temperatures sufficient to decompose the complex, and the unsaturated hydrocarbon separated from solution, e.g., by distillation. The temperature required for decomposition will vary according to the stability of the complex and varies from 0 to 200°C. Depending on the choice of solvent, elevated pressures may be required to maintain the inert organic solvent as a liquid. Decomposition products are either Cu metal and Cu(II) fluorinated acetylacetonate or Ag(I) fluorinated acetylacetonate depending on whether M above is Cu or Ag. The resultant mixture or solution is recycled and further contacted with feedstream thus reforming the metal complex and then re-heated to decomposition temperatures. This cyclical process is represented by the following equilibria:

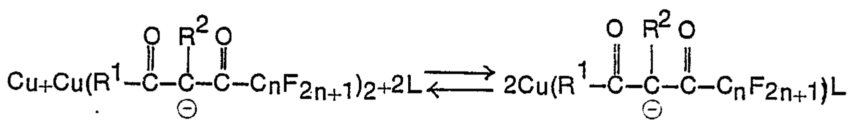

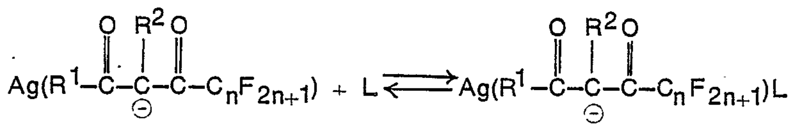

Gas feedstreams may contain other gases such as $N_2$, $H_2$, $CO_2$, alkanes, $H_2O$, $SO_2$, $SO_3$, and $NH_3$. The feedstream should preferably not, however, contain $H_2S$ or CO in amounts $> \sim 10$ vol.%, and $O_2$ amounts should preferably exceed about 10 vol.%. Liquid feedstreams may contain mixtures of organic solvents.

The process of the invention may be used to separate unsaturated hydrocarbons containing at least one non-aromatic unsaturation from gas and liquid feedstreams including those containing aromatic hydrocarbons. The process may also be used to separate optical isomers. If an optically active fluorinated acetylacetonate e.g., 3-trifluoroacetyl-d-camphor, is reacted with an unsaturated ligand which is a racemic mixture, a mixture of diastereoisomers is formed. By using known resolution techniques for separating the diastereoisomers, optically pure unsaturated ligands may be isolated. The invention is further illustrated by the following examples.

Example 1

A suspension of 1.43 g (0.01 mole) cuprous oxide in 75 ml tetrahydrofuran was stirred with 2.16 g (0.02 mole) of 1,5-cyclooctadiene in a 250 ml flask under nitrogen. A solution of 4.16 g (0.02 mole) 1,1,1,5,5,5-hexafluoroacetylacetone (hfacac) in 50 ml tetrahydrofuran was added dropwise over a 30 minute period. Red $Cu_2O$ gradually dissolved forming a clear yellow solution. The solution was filtered to remove any remaining solids and the solvent was then removed on a rotary evaporator. Cu(1,5-COD)(hfacac) was obtained as bright yellow crystals which could be purified by recrystallization from hexane. The product was characterized by IR and NMR spectroscopy and elemental analysis.

Example 2

A suspension of 10 mmole $Cu_2O$ in 100 ml tetrahydrofuran was prepared and 20 mmole hexafluoroacetylacetone was added to this mixture. After stirring, a gaseous mixture with the approximate composition 40% $H_2$, and 60% $C_2H_4$ was bubbled through the solution at room temperature and a complex was formed with an ethylene:Cu(I) salt ratio of 1:1. Analysis of the gas mixture over the solution indicated an essentially complete removal of ethylene.

In order to recover ethylene, $N_2$ was passed through the solution. Alternatively, the solution was heated to 50°C until ethylene evolution ceased.

Example 3

The procedure of Example 1 was repeated except that tetrahydrofuran is replaced with a solvent mixture of 90 ml of tetrahydrofuran and 10 ml water. Identical results were obtained showing that the absorption of ethylene is not appreciably affected by relatively large amounts of water.

Example 4

The procedure of Example 1 was repeated except that the gaseous mixture bubbled through the Cu(I) salt solution contained about 50% propane and 50% propylene. An analysis of the solution and gas mixture over the solution indicated the formation of a 1:1 propylene:Cu(I) complex and the nearly complete removal of propylene from the gas. This demonstrates that the absorption of olefins can take place in the presence of relatively large amounts of alkanes.

Example 5

A suspension of 10 mmole $Cu_2O$ in 100 ml methylene chloride was prepared and 20 mmole hexafluoroacetylacetone added to the mixture. A gas stream containing approximately 50% ethylene and 50% nitrogen was passed through the solution forming the 1:1 ethylene:Cu(I) salt. Heating the mixture to reflux resulted in the liberation of the ethylene and the formation of copper metal and copper II hexafluoracetylacetone. This mixture was cooled to room temperature and again exposed to the ethylene-nitrogen gas mixture which resulted in the formation of the 1:1 ethylene:Cu(I) salt. Heating to reflux again liberates ethylene and this cyclic process can be repeated indefinitely.

Examples 6—45

The examples shown in the following table further illustrate the types of unsaturated ligands which can be removed from feedstreams and the particular compelxes formed thereby.

TABLE I

| Ex. No. | Ligand (mmol) | -diketone (mmol) | Metal Oxide (mmol) | Solvent | Compound Formed |
|---|---|---|---|---|---|
| 6 | 1,5-cyclooctadiene (COD) (9.0) | thenoyltrifluoroacetone (TTA) (14.0) | $Cu_2O$ (9.0) | $CH_2Cl_2$ | Cu(COD)TTA |
| 7 | 1,5-cyclooctadiene (20.0) | hexafluoroacetylacetone (hfacac) (18.0) | $Cu_2O$ (11.0) | $C_6H_5CH_3$ | Cu(COD)hfacac |
| 8 | 1,3-butadiene (large excess) | hexafluoroacetylacetone (14.0) | $Cu_2O$ (7.0) | THF | $Cu(C_4H_6)$hfacac* |
| 9 | Diphenylacetylene (9.65) | hexafluoroacetylacetone (4.81) | $Cu_2O$ (3.0) | $CH_2Cl_2$ | (Cu(ØC≡CØ) )$_2$hfacac |
| 10 | Diphenylacetylene (9.65) | trifluoroacetylacetone (tfacac) (4.80) | $Cu_2O$ (3.0) | $CH_2Cl_2$ | (Cu(ØC≡CØ) )$_2$tfacac |
| 11 | 1,5-cyclooctadiene (20.0) | trifluoroacetylacetone (18.0) | $Cu_2O$ (11.0) | $CH_2Cl_2$ | Cu(COD)tfacac |
| 12 | Bicyclo[2.2.1] hepta-2,5-diene (22.0) | hexafluoroacetylacetone (21.0) | $Cu_2O$ (11.0) | $CH_2Cl_2$ | [Cu(hfacac)]$_2$ 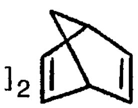 |
| 13 | cyclohexylisonitrile (13.0) | hexafluoroacetylacetone (6.0) | $Cu_2O$ (3.0) | $CH_2Cl_2$ | Cu(C ≡ N 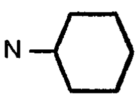 ) hfacac |
| 14 | Bicyclo[2.2.1]-2-heptene (18.0) | hexafluoroacetylacetone (17.0) | $Cu_2O$ (11.0) | $CH_2Cl_2$ | Cu ( 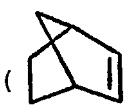 ) hfacac |
| 15 | 1,3,5,7-cyclo-octatetraene (COT) (7.5) | trifluoroacetylacetone (7.1) | $Cu_2O$ (4.0) | $CH_2Cl_2$ | Cu(COT)tfacac |
| 16 | 1,3,5,7-cyclo-octatetraene (3.6) | trifluoroacetylacetone (7.2) | $Cu_2O$ (4.0) | $CH_2Cl_2$ | [Cu(tfacac)]$_2$COT |

TABLE I (Continued)

| Ex. No. | Ligand (mmol) | –diketone (mmol) | Metal Oxide (mmol) | Solvent | Compound Formed |
|---|---|---|---|---|---|
| 17 | 2-hexyne (5.0) | hexafluoroacetylacetone (4.3) | $Cu_2O$ (2.5) | $CH_2Cl_2$ | $Cu(CH_3C{\equiv}CC_3H_7)$hfacac |
| 18 | styrene (11.5) | hexafluoroacetylacetone (9.61) | $Cu_2O$ (5.00) | $CH_2Cl_2$ | $Cu(CH_2{=}CH{-}Ø)$hfacac |
| 19 | isoprene (15.0) | hexafluoroacetylacetone (7.3) | $Cu_2O$ (4.0) | $CH_2Cl_2$ | $Cu(CH_2{=}C(CH_3){-}CH{=}CH_2)$hfacac |
| 20 | ethylene (large excess) | hexafluoroacetylacetone (6.9) | $Cu_2O$ (3.5) | $CH_2Cl_2$ | $Cu(CH_2{=}CH_2)$hfacac* |
| 21 | 2,8-decadiyne (15.0) | hexafluoroacetylacetone (14.0) | $Cu_2O$ (8.0) | $CH_2Cl_2$ | $[Cu(hfacac)]_2CH_3C{\equiv}C{-}(CH_2)_4C{\equiv}CCH_3$ |
| 22 | 1,5-cyclooactadiene (4.5) | 3-trifluoroacetyl-d-camphor (TAC) (4.0) | $Cu_2O$ (2.0) | $CH_2Cl_2$ | Cu(COD) (TAC) |
| 23 | cyclohexene (27.0) | hexafluoroacetylacetone (12.0) | $Cu_2O$ (7.0) | $CH_2Cl_2$ | Cu ( 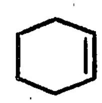 ) hfacac |
| 24 | Bicyclo[2.2.1]-2-heptene (14.0) | trifluoroacetylacetone (13.0) | $Cu_2O$ (6.5) | $CH_2Cl_2$ | Cu ( 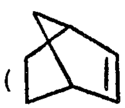 ) tfacac |
| 25 | cyclohexylisonitrile (16.0) | trifluroacetylacetone (8.2) | $Cu_2O$ (15.0) | $CH_2Cl_2$ | Cu(C ≡ N – 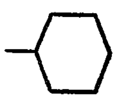 ) tfacac |
| 26 | phenylacetylene (15.0) | hexafluoroacetylacetone (15.0) | $Cu_2O$ (8.0) | $CH_2Cl_2$ | $Cu(ØC{\equiv}CH)$ hfacac |
| 27 | cyclooctene (COE) (9.07) | hexafluoroacetylacetone (9.0) | $Cu_2O$ (4.6) | $CH_2Cl_2$ | Cu(COE)hfacac |

TABLE I (Continued)

| Ex. No. | Ligand (mmol) | –diketone (mmol) | Metal Oxide (mmol) | Solvent | Compound Formed |
|---|---|---|---|---|---|
| 28 | propene (large excess) | hexafluoroacetylacetone (6.9) | $Cu_2O$ (3.5) | $CH_2Cl_2$ | $Cu(CH_3CH{=}CH_2)$hfacac* |
| 29 | 1-decene (24.0) | hexafluoroacetylacetone (24.0) | $Cu_2O$ (14.0) | $CH_2Cl_2$ | $Cu(CH_2{=}CH{-}C_8H_{17})$hfacac |
| 30 | 3-methylcyclohexene (15) | hexafluoroacetylacetone (9.0) | $Cu_2O$ (5.0) | $CH_2Cl_2$ | Cu($CH_3$ 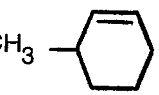 )hfacac |
| 31 | 1,3,5,7-cyclo-octatetraene (7.5) | hexafluoroacetylacetone (7.2) | $Cu_2O$ (4.0) | $CH_2Cl_2$ | Cu(COT)hfacac |
| 32 | 1,3,5,7-cyclo-octatetraene (3.6) | hexafluoroacetylacetone (7.2) | $Cu_2O$ (4.0) | $CH_2Cl_2$ | $[Cu(hfacac)]_2COT$ |
| 33 | (+)–α-pinene (7.5) | hexafluoroacetylacetone (7.4) | $Cu_2O$ (4.0) | $CH_2Cl_2$ | Cu(α-pinene) hfacac |
| 34 | 3-methyl-cyclo-hexene (8.0) | 3-trifluoroacetyl-d-camphor (6.0) | $Cu_2O$ (3.5) | $CH_2Cl_2$ | Cu($CH_3$ 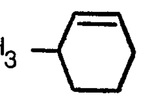 ) TAC |
| 35 | d,1-α-pinene (8.0) | 3-trifluoroacetyl-d-camphor (6.0) | $Cu_2O$ (3.5) | $CH_2Cl_2$ | Cu(d-α-pinene)TAC + Cu(1-α-pinene)TAC, mixed diastereomers |
| 36 | 1,5-cyclooctadiene (1.12) | hexafluoroacetylacetone (1.12) | $Ag_2O$ (0.56) | $CH_2Cl_2$ | Ag(COD)hfacac |
| 37 | ethylene (large excess) | hexafluoroacetylacetone (4.28) | $Ag_2O$ (2.16) | $CH_2Cl_2$ | $Ag(CH_2{=}CH_2)$hfacac* |
| 38 | diphenylacetylene (8.64) | hexafluoroacetylacetone (4.30) | $Ag_2O$ (2.16) | $CH_2Cl_2$ | Ag(ØC≡CØ) hfacac |

TABLE I (Continued)

| Ex. No. | Ligand (mmol) | –diketone (mmol) | Metal Oxide (mmol) | Solvent | Compound Formed |
|---|---|---|---|---|---|
| 39 | cyclooctene (8.64) | hexafluoroacetylacetone (8.64) | $Ag_2O$ (4.37) | $CH_2Cl_2$ | Ag(COE)hfacac |
| 40 | propylene (large excess) | hexafluoroacetylacetone (4.28) | $Ag_2O$ (2.16) | $CH_2Cl_2$ | $Ag(CH_3CH{=}CH_2)hfacac$ |
| 41 | 1-decene (4.4) | hexafluoroacetylacetone (4.3) | $Ag_2O$ (2.2) | $CH_2Cl_2$ | $Ag(CH_2{=}CHC_8H_{17})hfacac$ |
| 42 | 1,3-butadiene (large excess) | hexafluoroacetylacetone (4.3) | $Ag_2O$ (2.16) | $CH_2Cl_2$ | $Ag(CH_2{=}CH{-}CH{=}CH_2)hfacac$* |
| 43 | bicyclo[2.2.1]-2-heptene (4.4) | hexafluoroacetylacetone (4.3) | $Ag_2O$ (2.2) | $CH_2Cl_2$ | Ag ( 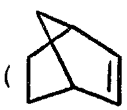 ) hfacac |
| 44 | bicyclo[2.2.1]-hepta-2,5-diene (4.4) | hexafluoroacetylacetone (4.3) | $Ag_2O$ (2.2) | $CH_2Cl_2$ | [Ag(hfacac)]$_2$ 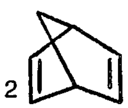 |
| 45 | diethyl maleate | hexafluoroacetylacetone (20.0) | $Cu_2O$ (10.0) | none | $Cu(C_2H_5O\overset{O}{\overset{\|}{C}}{-}CH{=}CH{-}\overset{O}{\overset{\|}{C}}{-}OC_2H_5)hfacac$ |

*Not stable at room temperature.

**Claims**

1. A complex of the formula

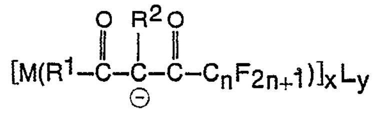

where M is Cu(I) or Ag(I); $R^1$ is $C_1$—$C_6$ fluoralkyl, $C_1$—$C_8$ alkyl, $C_4$—$C_6$ heterocycle containing O, S or N or $C_6$—$C_{10}$ aryl; $R^2$ is H or $C_1$—$C_6$ alkyl with the proviso that $R^1$ and $R^2$ together with the carbon atoms to which they are attached may be joined together to form a $C_6$ ring or a 1,7,7-trimethyl-2-oxobicyclo[2,2,1]hept-3-yl radical; L is an unsaturated hydrocarbon ligand containing at least one ethylenic, acetylenic or isonitrilic unsaturation; x and y are 1 or 2; and n is an integer from 1 to 8 provided that when M is Ag, if L is an olefin, said olefin must be a linear olefin.

2. A complex according to claim 1 wherein L is an (a) alkene of the formula

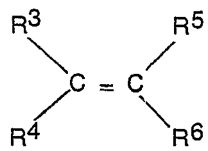

where each $R^3$—$R^6$ is independently H; $C_1$—$C_{30}$ aliphatic with the proviso that any combination of $R^3$, $R^4$, $R^5$ and $R^6$ may be joined together to form at least one $C_4$—$C_{14}$ cycloaliphatic ring; —C≡N; $C_6$—$C_{10}$ aryl; $C_7$—$C_{14}$ araliphatic;

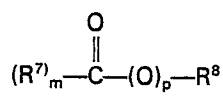

where m and p are 0 or 1, $R^7$ is $C_1$—$C_{20}$ aliphatic, $R^8$ is H, $C_1$—$C_{10}$ aliphatic or $C_6$—$C_{10}$ aryl with the proviso that adjacent

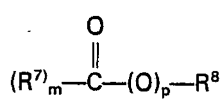

may be joined together to form a $C_4$—$C_{16}$ anhydride; (b) alkyne of the formula $R^9$—C≡C—$R^{10}$ where $R^9$ and $R^{10}$ are independently H; $C_1$—$C_{30}$ aliphatic; $C_6$—$C_{10}$ aryl or $C_7$—$C_{14}$ araliphatic; or (c) isonitrile of the formula $R^{11}$—N≡C where $R^{11}$ is $C_1$—$C_{20}$ aliphatic; $C_3$—$C_{10}$ cycloaliphatic; $C_7$—$C_{20}$ araliphatic or $C_6$—$C_{10}$ aryl.

3. A complex according to claim 2 wherein $R^3$—$R^6$ are independently H, $C_1$—$C_{15}$ aliphatic, $C_5$—$C_{12}$ cycloaliphatic, $C_6$—$C_{10}$ aryl or $C_7$—$C_{14}$ araliphatic.

4. A complex according to claim 2 wherein $R^1$ is $CF_3$, $CH_3$ or

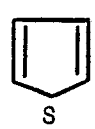

$R^2$ is H, n is 1, x and y are 1 and L is

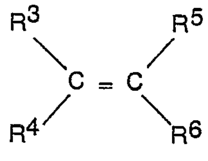

wherein $R^3$, $R^4$, $R^5$ and $R^6$ are as defined in claim 2.

5. A complex according to claim 1 wherein $R^1$ is $C_1$—$C_3$ fluoroalkyl, $C_1$—$C_6$ alkyl, $C_6$—$C_{10}$ aryl or $C_4$ or $C_5$ heterocycle containing O, S or N and preferably $CF_3$.

6. A complex according to claim 1 wherein $R^2$ is H and n is 1.

7. A process for preparing a complex according to any one of the preceding claims or for removing unsaturated hydrocarbons containing at least one ethylenic, acetylenic or isonitrilic unsaturation from feedstreams which comprises contacting the feedstream with at least one of $Cu_2O$ or $Ag_2O$ and a fluorinated acetylacetonate of the formula

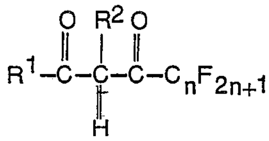

where $R^1$, $R^2$ and n are as defned in claim 1, in the presence of an inert organic solvent.

8. A process for preparing a complex according to any one of claims 1—6 or for removing unsturated hydrocarbons containing at least one ethylenic, acetylenic or isonitrilic unsaturation from feedstreams which comprises contacting the feedstream with Cu metal and a Cu (II) fluorinated acetylacetonate of the formula

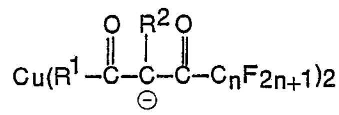

or a Ag(I) fluorinated acetylacetonate of the formula

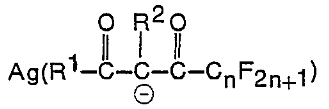

where $R^1$, $R^2$ and n are as defined in claim 1, in an inert organic solvent.

9. A process according to either of claims 7 and 8 wherein the amounts of $Cu_2O$, $Ag_2O$ and fluorinated acetylacetonate or the amounts of Cu metal and Cu(II) fluorinated acetylacetonate or Ag(I) fluorinated acetylacetonate are from 0.001 to 5 M.

10. A process according to any one of claims 7 to 9 wherein the temperature is from −100 to +100°C.

11. A process according to any one of claims 7 to 10 wherein the solution containing Cu(I) or Ag(I) complex with fluorinated acetylacetonate and unsaturated hydrocarbon having at least one ethylenic, acetylenic or isonitrilic unsaturation is heated to a temperature sufficient to decompose the complex, and the solution is recycled and further contacted with the feedstream.

12. A process according to any one of claims 7 to 11 wherein the unsaturated hydrocarbon is (a) an alkene of the formula

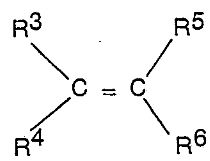

where each $R^3$—$R^6$ is as defined in claim 2.

**Revendications**

1. Complexe de formule

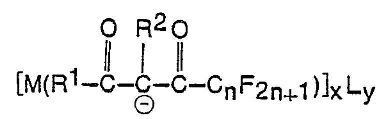

où M est Cu(I) ou Ag(I); $R^1$ est un radical fluoralkyle en $C_1$ à $C_6$, alkyle en $C_1$ à $C_8$, hétérocyclique en $C_4$ à $C_6$ contenant O, S ou N, ou aryle en $C_6$ à C10; $R^2$ est H ou un radical alkyle en $C_1$ à $C_6$, à la condition que $R^1$ et $R^2$ puissent, avec les atomes de carbone auxquels ils sont fixés, être réunis pour former un cycle en $C_6$ ou un radical 1,7,7-triméthyl-2-oxo-bicyclo[2,2,1]hept-3-yl, L est un ligand hydrocarboné insaturé contenant au moins une insaturation éthylénique, acétylénique ou isonitrilique; x et y sont 1 ou 2; et n est un entier de 1 à 8, à la condition que, quand M est Ag, si L est une oléfine, cette oléfine doit être une oléfine linaire.

2. Complexe selon la revendication 1, dans lequel L est (a) un alcène de formule

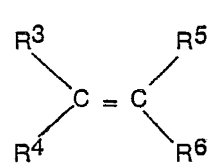

où chaque radical $R^3$ à $R^6$ est, indépendamment des autres, H; un radical aliphatique en $C_1$ à $C_{30}$ à la

condition que toute combinaison de $R^3$, $R^4$, $R^5$ et $R^6$ puisse être réunie pour former au moins un cycle aliphatique en $C_4$ à $C_{14}$; —C≡N; aryle en $C_6$ à $C_{10}$; un radical araliphatique en $C_7$ à $C_{14}$;

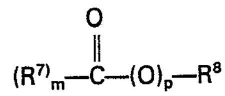

où m et p sont 0 ou 1, $R^7$ est un radical aliphatique en $C_1$ à $C_{20}$, $R^8$ est H, un radical aliphatique en $C_1$ à $C_{10}$ ou aryle en $C_6$ à $C_{10}$ à la condition que les groupes

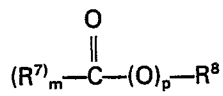

adjacent puissent être réunis pour former un anhydride en $C_4$ à $C_{16}$; (b) un alcyne de formule $R^9$—C≡C—$R^{10}$ où $R^9$ et $R^{10}$ sont, indépendamment l'un de l'autre, H; un radical aliphatique en $C_1$ à $C_{30}$; aryle en $C_6$ à $C_{10}$ ou araliphatique en $C_7$ à $C_{14}$; ou (c) un radical isonitrile de formuel $R^{11}$—N≡C, où $R^{11}$ est un radical aliphatique en $C_1$ à $C_{20}$; cycloaliphatique en $C_3$ à $C_{10}$; araliphatique en $C_7$ à $C_{20}$ ou aryle en $C_6$ à $C_{10}$.

3. Complexe selon la revendication 2, dans lequel $R^3$ à $R^6$ sont, indépendamment les uns des autres, H, un radical aliphatique en $C_1$ à $C_{15}$, cycloaliphatique en $C_5$ à $C_{12}$, aryle en $C_6$ à $C_{10}$ ou araliphatique en $C_7$ à $C_{14}$.

4. Complexe selon la revendication 2, dans lequel $R^1$ est $CF_3$, $CH_3$ ou

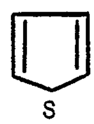

$R^2$ est H, n est 1, x et y sont 1 et L est

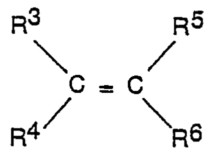

où $R^3$, $R^4$, $R^5$ et $R^6$ sont comme il a été défini dans la revendication 2.

5. Complexe selon la revendication 1, dans lequel $R^1$ est un radical fluoralkyle en $C_1$ à $C_3$, alkyle en $C_1$ à $C_6$, aryle en $C_6$—$C_{10}$ ou un hétérocycle en $C_4$ ou $C_5$ contenant O, S ou N, et de préférence $CF_3$.

6. Complexe selon la revendication 1, dans lequel $R^2$ est H et n vaut 1.

7. Procédé pour la préparation d'un complexe selon l'une quelconque des revendications précédentes ou pour éliminer de flux de charge des hydrocarbures insaturés contenant au moins une insaturation éthylénique, acétylénique ou isonitrilique, qui consiste à mettre en contact le flux de charge avec au moins l'un des composés $Cu_2O$ ou $Ag_2O$ et un acétylacétonate fluoré de formule

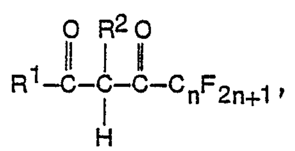

où $R^1$, $R^2$ et n sont comme il a été défini dans la revendication 1, en présence d'un solvant organique inerte.

8. Procédé pour la préparation d'un complexe selon l'une quelconque des revendications 1 à 6 ou pour extraire de flux de charge des hydrocarbures insaturés contenant au moins une insaturation éthylénique, acétylénique ou isonitrilique, qui consiste à mettre en contact le flux de charge avec du Cu métallique et un acétylacétonate fluoré de Cu(II) de formule

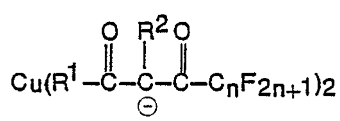

ou un acétylacétonate fluoré de Ag(I) de formule

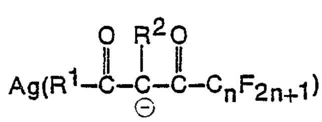

où $R^1$, $R^2$ et n sont comme il a été défini dans la revendication 1, dans un solvant organique inerte.

9. Procédé selon l'une des revendications 7 ou 8, dans lequel les quantités de $Cu_2O$, $Ag_2O$ et d'acétylacétonate fluoré, ou les quantités de Cu métallique et d'acétylacétonate fluoré de Cu(II) ou d'acétylacétonate fluoré de Ag(I) sont comprises entre 0,001 et 5 M.

10. Procédé selon l'une quelconque des revendications 7 à 9, dans lequel la température est comprise entre −100 et +100°C.

11. Procédé selon l'une quelconque des revendications 7 à 10, dans lequel la solution contenant le complexe de Cu(I) ou de Ag(I) avec l'acétylacétonate fluoré et un hydrocarbure insaturé possédant au moins une insaturation éthylénique, acétylénique ou isonitrilique, est chauffée à une température suffisante pour décomposer le complexe, et la solution est recyclée et de nouveau mise en contact avec le flux de charge.

12. Procédé selon l'une quelconque des revendications 7 à 11, dans lequel l'hydrocarbure insaturé est (a) un alcène de formule

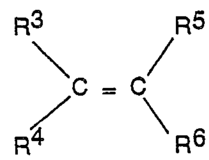

où chaque $R^3$ à $R^6$ est comme il a été défini dans la revendication 2.

**Patentansprüche**

1. Komplex mit der Formel

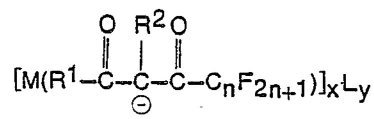

in der M Cu(I) oder Ag(I) ist, $R^1$ ein $C_1$—$C_6$-Fluoralkylrest, $C_1$—$C_8$-Alkylrest, ein O, S oder N enthaltender heterocyclischer $C_4$—$C_6$-Rest oder ein $C_6$—$C_{10}$-Alkylrest und $R^2$ H oder ein $C_1$—$C_6$-Alkylrest ist mit der Maßgabe, daß $R^1$ und $R^2$ zusammen mit den Kohlenstoffatomen, an die sie gebunden sind, zu einem $C_6$-Ring oder einem 1,7,7-Trimethyl-2-oxobicyclo[2,2,1]hept-3-yl-Rest miteinander verbunden sein können, L ein ungesättigter Kohlenwasserstoffligand ist, der mindestens eine ethylenische, acetylenische oder isonitrilische Ungesättigtheit enthält, x und y 1 oder 2 sind und n eine ganze Zahl von 1 bis 8 ist, wobei dann, wenn M Ag und L ein Olefin ist, dieses Olefin ein lineares Olefin sein muß.

2. Komplex nach Anspruch 1, dadurch gekennzeichnet, daß L

(a) ein Alken mit der Formel

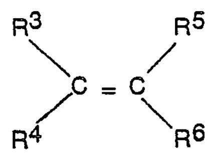

in der jeder $R^3$ bis $R^6$ unabhängig H, ein aliphatischer $C_1$—$C_{30}$-Rest mit der Maßgabe, daß jede Kombination von $R^3$, $R^4$, $R^5$ und $R^6$ unter Bildung mindestens eines cycloaliphatischen $C_4$—$C_{14}$-Ringes verbunden sein kann, —C≡N, ein $C_6$—$C_{10}$-Arylrest, ein araliphatischer $C_7$—$C_{14}$-Rest oder $(R^7)_m$—C—$(O)_p$—$R^8$ ist, wobei m und p O oder 1 sind, $R^7$ ein aliphatischer $C_1$—$C_{20}$-Rest ist, $R^8$ H, ein aliphatischer $C_1$—$C_{10}$-Rest oder ein $C_6$—$C_{10}$-Arylrest mit der Maßgabe ist, daß benachbarte

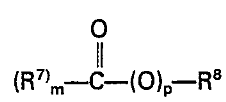

unter Bildung eines $C_4$—$C_{16}$-Anhydrids verbunden sein können, (b) ein Alkin mit der Formel $R^9$—C≡C—$R^{10}$, in der $R^9$ und $R^{10}$ unabhängig H, ein aliphatischer $C_1$—$C_{30}$-Rest, ein $C_6$—$C_{10}$-Arylrest oder araliphatische $C_7$—$C_{14}$-Reste sind, oder (c) ein Isonitril mit der Formel $R^{11}$—N≡C ist, in der $R^{11}$ ein aliphatischer $C_1$—$C_{20}$-Rest, ein cycloaliphatischer $C_3$—$C_{10}$-Rest, ein araliphatischer $C_7$—$C_{20}$ oder ein $C_6$—$C_{10}$-Arylrest ist.

3. Komplex nach Anspruch 2, dadurch gekennzeichnet, daß $R^3$ bis $R^9$ unabhängig H, ein aliphatischer $C_1$—$C_{15}$-Rest, ein cycloaliphatischer $C_5$—$C_{12}$-Rest, ein $C_6$—$C_{10}$-Arylrest oder ein araliphatischer $C_7$—$C_{14}$-Rest sind.

4. Komplex nach Anspruch 2, dadurch gekennzeichnet, daß $R^1$ $CF_3$, $CH_3$ oder

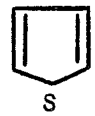

ist, $R^2$ H ist, n 1 ist, x und y 1 sind und L

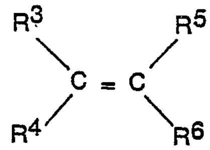

ist, wobei $R^3$, $R^4$, $R^5$ und $R^6$ die in Anspruch 2 angegebene Bedeutung haben.

5. Komplex nach Anspruch 1, dadurch gekennzeichnet, daß $R^1$ ein $C_1$—$C_3$-Fluoralkylrest, ein $C_1$—$C_6$-Alkylrest, ein $C_6$—$C_{10}$-Arylrest oder ein O, S oder N enthaltender heterocyclischer $C_4$- oder $C_5$-Rest ist und vorzugsweise $CF_3$ ist.

6. Komplex nach Anspruch 1, dadurch gekennzeichnet daß $R^2$ H ist und n 1 ist.

7. Verfahren zur Herstullung eines Komplexes gemäß einem der vorangehenden Ansprüche oder zur Entfernung ungesättigter Kohlenwasserstoff, die mindestens eine ethylenische, acyetylenische oder isonitrilische Ungesättigtkeit enthalten, aus Einsatzproduktströmen, dadurch gekennzeichnet, daß der Einsatzproduktstrom mit $Cu_2O$ und/oder $Ag_2O$ und einem fluorierten Acetylacetonat mit der Formel

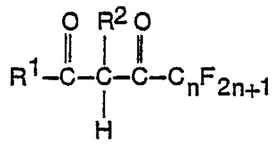 ,

wobei $R^1$, $R^2$ und n die in Anspruch 1 angegebene Bedeutung besitzen, in Gegenwart eines inerten organischen Lösungsmittels kontaktiert wird.

8. Verfahren zur Herstellung eines Komplexes gemäß einem der Ansprüche 1 bis 6 oder zur Entfernung von ungesättigten Kohlenwasserstoffen, die mindestens eine ethylenische, acetylenische oder isonitrilische Ungesättigtheit enthalten, aus Einsatzproduktströmen, dadurch gekennzeichnet, daß der Einsatzproduktstrom mit Cu-Metall und einem fluorierten Cu(II)-acetylacetonat mit der Formel

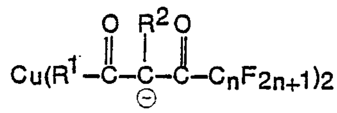

oder einem fluorierten Ag(I)-acetylacetonat mit der Formel

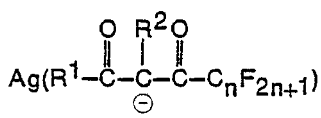 ,

wobei $R^1$, $R^2$ und n die in Anspruch 1 angegebene Bedeutung besitzen, in einem inerten organischen Lösungsmittel kontaktiert wird.

9. Verfahren nach Anspruch 7 oder 8, dadurdch gekennzeichnet, daß die Mengen an $Cu_2O$, $Ag_2O$ und fluoriertem Acetylacetonat oder die Mengen an Cu-Metall und fluoriertem Cu(II)-acetylacetonat oder fluoriertem Ag(I)-acetylacetonat 0,001 bis 5 M betragen.

10. Verfahren nach einem der Ansprüche 7 bis 9, dadurch gekennzeichnet, daß die Temperatur von $-100$ bis $+100$°C beträgt.

11. Verfahren nach einem der Ansprüche 7 bis 10, dadurch gekennzeichnet, daß die Lösung, die Cu(I)- oder Ag(I)-Komplex mit fluoriertem Acetylacetonat und einem ungesättigten Kohlenwasserstoff mit mindestens einer ethylenischen, acetylenischen oder isonitrilischen Ungesättigtheit enthält, auf eine für die Zersetzung des Komplexes ausreichende Temperatur erwärmt wird und die Lösung zurückgeführt und wieder mit dem Einsatzproduktstrom kontaktiert wird.

12. Verfahren nach einem der Ansprüche 7 bis 11, dadurch gekennzeichnet, daß der ungesättigte Kohlenwasserstoff (a) ein Alken mit der Formel

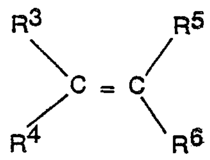

ist, in der jedes $R^3$ bis $R^6$ die in Anspruch 2 gegebene Bedeutung besitzt.